(19) Europäisches Patentamt
European Patent Office
Office européen des brevets

(11) Veröffentlichungsnummer: **0 098 577**
A2

(12) # EUROPÄISCHE PATENTANMELDUNG

(21) Anmeldenummer: **83106536.2**

(22) Anmeldetag: **05.07.83**

(51) Int. Cl.³: **A 61 K 45/06**, A 61 K 31/44, A 61 K 31/47, A 61 K 31/435, A 61 K 31/445, A 61 K 31/495, A 61 K 31/50 // (A61K31/44, 31/435)

(30) Priorität: **07.07.82 DE 3225367**

(43) Veröffentlichungstag der Anmeldung: **18.01.84 Patentblatt 84/3**

(84) Benannte Vertragsstaaten: **DE FR GB IT**

(71) Anmelder: **HOECHST AKTIENGESELLSCHAFT, Postfach 80 03 20, D-6230 Frankfurt am Main 80 (DE)**

(72) Erfinder: **Schrinner, Elmar, Dr., Hedwigstrasse 2, D-6200 Wiesbaden (DE)**

(54) **Arzneimittel zur Behandlung von infektiös bedingten Diarrhöen.**

(57) Arzneimittel zur Behandlung von Diarrhöen, gekennzeichnet durch einen Gehalt an a) einer im Darmlumen antiseptisch wirkenden Substanz und b) einer systemisch antibakteriell wirkenden Substanz.

EP 0 098 577 A2

ACTORUM AG

## Arzneimittel zur Behandlung von infektiös bedingten Diarrhöen

Zur Behandlung infektiös bedingter Diarrhöen werden meist antibakteriell, aber auch adstringierend oder detoxifizierend wirkende Substanzen verwendet. Bei derartigen Diarrhöen ist die Darmflora verändert; sie ist mit pathogenen Keimen besiedelt. Eine Abtötung dieser pathogenen Keime ist die wichtigste therapeutische Maßnahme. Daneben ist bei akuten Diarrhöeformen auch mit einer schubweisen Invasion der Erreger in den Kreislauf zu rechnen. Diese Erreger können nicht durch die gebräuchlichen nichtresorbierbaren Antidiarrhoica erfaßt werden.

Es ist bekannt, daß die Acridinderivate Trypaflavin und Ethacridinlactat ebenso wie die Chinolinderivate Clioquinol und Halquinol, amöbicid und bakteriostatisch wirken und deshalb gegen infektiöse Diarrhöen verwendet werden können. Andererseits ist bekannt, daß Verbindungen aus der Reihe der Nalidixinsäure, sowohl Chinolinon-Verbindungen als auch Naphthyridin-Verbindungen, ein breites antibakterielles Wirkungsspektrum aufweisen.

Gegenstand der Erfindung sind Arzneimittel zur Behandlung von infektiös bedingten Diarrhöen, wie sie in den Patentansprüchen definiert sind. Grundgedanke der Erfindung ist, ein neues Arzneimittel zur Behandlung solcher Diarrhöen zu schaffen, welches aus einer Verbindung besteht, die im Darmlumen amöbicid und bakterizid bzw. bakteriostatisch wirkt, kombiniert mit einer anderen Verbindung, die im Verdauungstraktus resorbiert wird und systemisch antibakteriell wirkt. Dabei hat sich herausgestellt, daß gewisse Kombinationen solcher Verbindungen nicht nur kompatibel sind, sondern in gewissen Mischungsverhältnissen einen starken Synergismus gegen einen großen Teil von

infektiösen grampositiven und gramnegativen Keimen zeigen.

Diese sich überadditiv ergänzende antibakterielle Wirkung hat nicht nur eine Bedeutung für die Abtötung der Infektionserreger, sondern führt auch zu einer Verringerung des Risikos einer schnellen Resistenzentwicklung. Ferner kann bei individueller Dosierung mit einer Dosiseinsparung gerechnet werden. Somit führt der Synergismus beider Verbindungen auch zu einer geringeren Substanzbelastung des Organismus des Patienten sowie zu einer wirtschaftlicheren Therapie. Aus diesen Gründen eignen sich derartige Kombinationen besonders zur Therapie bakteriell bedingter Diarrhöen.

Zur Untersuchung der erfindungsgemäßen Kombination wurden mit typischen Vertretern der grampositiven und gramnegativen Keimgruppen in vitro Versuche durchgeführt. Mit der für Kombinationsversuche üblichen Methode, der sogenannten "Schachbrettmethode" (vgl. "Antibiotics in Laboratory Medicine", Editor Lorian, Baltimore London 1980, Seiten 300-309), wurden sowohl die minimalen Hemmkonzentrationen (MHK) als auch die Hemmgrenzen in den einzelnen Kombinationen bestimmt. Das Endergebnis wurde als FIC-Index (Fractional Inhibitory Concentration Index) ausgedrückt. Der FIC-Index berechnet sich nach folgender Formel:

$$\frac{A}{MHK_A} + \frac{B}{MHK_B} = FIC_A + FIC_B = \text{FIC-Index}$$

Hierbei ist A bzw. B die niedrigste Konzentration der Verbindungen A bzw. B, die in Kombination mit der Verbindung B bzw. A eine Hemmwirkung zeigt. Dieser Wert wird durch die MHK der Verbindung A bzw. B dividiert. Der so erhaltene FIC-Index gibt Aufschluß über die

Interaktionen von zwei Verbindungen in ihrer antibakteriellen Wirkung. FIC-Index-Zahlen, die kleiner als 1 sind, drücken eine synergistische Wirkung aus. Die mit dieser Methode ermittelten Resultate sind in den nachstehenden Tabellen zusammengestellt.

In den nachstehenden Tabellen sind in den ersten beiden Spalten die minimalen Hemmkonzentrationen der geprüften Verbindungen in Mikrogramm pro Milliliter aufgeführt. In einigen Fällen sind abweichende Konzentrationseinheiten angegeben, z.B. Nanogramm /ng/ oder Milligramm /mg/ pro Milliliter. Wenn ein FIC-Index nicht angegeben wurde, so bedeutet dies, daß aufgrund der Unwirksamkeit des einen Wirkstoffes keine Grenze zu ermitteln war.

TABELLE 1

Antibakterielle Wirkung in vitro von Ethacridinlactat (EAL) und 5-Äthyl-5,8-dihydro-8-oxofuran(3,2-b)(1,8)-naphthyridin-7-carbonsäure (OFNC)

| Bakterien-Stamm | MHK in µg/ml | | |
|---|---|---|---|
| | EAL | OFNC | FIC-Index |
| Staph. aureus SG 511 | 3,125 | 0,391 | 2,0 |
| Micrococcus luteus ATCC 9341 | 6,25 | 31,25 | 2,0 |
| Strept. pyogenes A 77 | 0,313 | 125,0 | 2,0 |
| faecium D | 15,625 | 31,25 | 0,375 |
| Bacillus subtilis ATCC 6633 | 1,563 | 48,828 ng | 1,0 |
| Pseud. aeruginosa ATCC 9027 | 7,813 | 3,906 | 0,313 |
| aeruginosa NCTC 10701 | 15,625 | 1,953 | 0,156 |
| aeruginosa 1771 m | 15,625 | 1,953 | 0,094 |
| E. coli 0 55 | 6,25 | 0,125 | 0,25 |
| 0 78 | 15,625 | 0,125 | 0,188 |
| 0 86 | 15,625 | 62,5 ng | 0,375 |
| TEM | 15,625 | 0,156 | 0,156 |
| Salmonella paratyphi A | 12,5 | 39,063 ng | 0,047 |
| typhimurium | 25,0 | 78,125 ng | 0,156 |
| oranienburg | 62,5 | 78,125 ng | 0,079 |
| Klebsiella pneumoniae A 9977 | 125,0 | 78,125 ng | 0,071 |
| Enterobacter cloacae | 12,5 | 0,781 | 0,032 |
| Hafnia alvei ATCC 11604 | 62,5 | 39,063 ng | 0,024 |
| Serratia marcescens A 20019 | 1,0 mg | 0,156 | 0,065 |
| marcescens A 20460 | > 1,0 mg | 0,625 | — |
| Proteus rettgeri 936 | 31,25 | 31,25 ng | 0,266 |

TABELLE 2

Antibakterielle Wirkung in vitro von Ethacridinlactat (EAL) und Nalidixinsäure sowie von deren Kombination

| Bakterien-Stamm | MHK in µg/ml | | FIC-Index |
|---|---|---|---|
| | EAL | Nalidixinsäure | |
| Staph. aureus SG 511 | 6,25 | 50,0 | 0,5 |
| Micrococcus luteus ATCC 9341 | 7,813 | > 1,0 mg | - |
| Strept. pyogenes A 77 | 0,313 | > 1,0 mg | - |
| faecium D | 7,813 | 500,0 | 2,0 |
| Bacillus subtilis ATCC 6633 | 3,125 | 6,25 | 1,0 |
| Pseud. aeruginosa ATCC 9027 | 15,625 | 500,0 | 0,281 |
| aeruginosa NCTC 10701 | 15,625 | 250,0 | 0,563 |
| aeruginosa 1771 m | 31,25 | 100,0 | 0,313 |
| E. coli 0 55 | 6,25 | 1,563 | 1,0 |
| 0 78 | 15,625 | 3,125 | 0,25 |
| 0 86 | 15,625 | 1,563 | 0,375 |
| TEM | 12,5 | 1,563 | 0,5 |
| Salmonella paratyphi A | 12,5 | 1,563 | 1,0 |
| typhimurium | 25,0 | 0,781 | 2,0 |
| oranienburg | 125,0 | 3,125 | 0,141 |
| Klebsiella pneumoniae A 9977 | 125,0 | 6,25 | 0,062 |
| Enterobacter cloacae | 62,5 | 6,25 | 0,126 |
| Hafnia alvei ATCC 11604 | 62,5 | 1,563 | 0,313 |
| Serratia marcescens A 20019 | > 1,0 mg | 1,563 | - |
| marcescens A 20460 | 125,0 | 6,25 | 0,188 |
| Proteus rettgeri 936 | 62,5 | 1,563 | 1,0 |

TABELLE 3

Antibakterielle Wirkung in vitro von Ethacridinlactat (EAL) und Miloxacin sowie deren Kombination

| Bakterien-Stamm | MHK in µg/ml | | FIC-Index |
|---|---|---|---|
| | EAL | Miloxacin | |
| Staph. aureus SG 511 | 6,25 | 6,25 | 1,0 |
| Micrococcus luteus ATCC 9341 | 7,813 | > 1,0 mg | - |
| Strept. pyogenes A 77 | 0,195 | > 1,0 mg | - |
| faecium D | 7,813 | > 1,0 mg | - |
| Bacillus subtilis ATCC 6633 | 1,563 | 0,781 | 1,0 |
| Pseud. aeruginosa ATCC 9027 | 12,5 | 25,0 | 0,188 |
| aeruginosa NCTC 10701 | 12,5 | 31,25 | 0,375 |
| aeruginosa 1771 m | 62,5 | 50,0 | 0,094 |
| E. coli 0 55 | 12,5 | 0,195 | 0,75 |
| 0 78 | 15,625 | 0,625 | 0,5 |
| 0 86 | 15,625 | 0,313 | 1,0 |
| TEM | 15,625 | 0,625 | 0,75 |
| Salmonella paratyphi A | 15,625 | 0,625 | 1,0 |
| typhimurium | 25,0 | 0,391 | 0,375 |
| oranienburg | 125,0 | 0,625 | 0,75 |
| Klebsiella pneumoniae A 9977 | 125,0 | 0,625 | 1,0 |
| Enterobacter cloacae | 62,5 | 2,5 | 0,281 |
| Hafnia alvei ATCC 11604 | 62,5 | 0,625 | 0,375 |
| Serratia marcescens A 20019 | > 1,0 mg | 0,625 | - |
| marcescens A 20460 | 1,0 mg | 0,625 | 0,25 |
| Proteus rettgeri 936 | 62,5 | 0,625 | 2,0 |

TABELLE 4

Antibakterielle Wirkung in vitro von Ethacridinlactat (EAL) und Norfloxacin sowie von deren Kombination

| Bakterien-Stamm | MHK in µg/ml | | FIC-Index |
|---|---|---|---|
| | EAL | Norfloxacin | |
| Staph. aureus SG 511 | 6,25 | 0,313 | 2,0 |
| Micrococcus luteus ATCC 9341 | 6,25 | 12,5 | 2,0 |
| Strept. pyogenes A 77 | 0,391 | 2,5 | 2,0 |
| faecium D | 12,5 | 1,25 | 1,0 |
| Bacillus subtilis ATCC 6633 | 1,563 | 0,156 | 0,625 |
| Pseud. aeruginosa ATCC 9027 | 25,0 | 0,625 | 1,031 |
| aeruginosa NCTC 10701 | 25,0 | 0,625 | 2,0 |
| aeruginosa 1771 m | 62,5 | 0,625 | 1,0 |
| E. coli 0 55 | 12,5 | 39,063 ng | 1,0 |
| 0 78 | 15,625 | 0,156 | 0,625 |
| 0 86 | 15,625 | 39,063 ng | 0,5 |
| TEM | 15,625 | 39,063 ng | 2,0 |
| Salmonella paratyphi A | 15,625 | 19,531 ng | 1,0 |
| typhimurium | 50,0 | 19,531 ng | 1,0 |
| oranienburg | 62,5 | 78,125 ng | 1,0 |
| Klebsiella pneumoniae A 9977 | 125,0 | 0,313 | 0,375 |
| Enterobacter cloacae | 62,5 | 0,156 | 0,313 |
| Hafnia alvei ATCC 11604 | 62,5 | 19,531 ng | 0,375 |
| Serratia marcescens A 20019 | >1,0 mg | 78,125 ng | – |
| marcescens A 20460 | 500,0 | 0,156 | 0,188 |
| Proteus rettgeri 936 | 31,25 | 78,125 ng | 0,563 |

Die Versuche haben ergeben, daß eine Kombination von Ethacridinlactat mit 5-Äthyl-5,8-dihydro-8-oxofuran-(3,2-b)-(1,8)-naphthyridin-7-carbonsäure die kleinsten FIC-Indices ergaben und somit am stärksten synergistisch auf die Mehrzahl der geprüften Keime wirkten.

Synergistische Wirkungen zeigen insbesondere solche Mischungen, die dadurch gekennzeichnet sind, daß das Gewichtsverhältnis von einer im Darmlumen antiseptisch wirkenden Verbindung wie Ethacridinlactat, Trypaflavin, Clioquinol oder Halquinol zu einer systemisch antibakteriell wirkenden Verbindung der Formeln I bis XVII, wie sie im einzelnen in den Patentansprüchen aufgeführt sind, zwischen 1:0,4 und 1:5 liegt.

Beispiel 1

| Tabletten | 1 Tablette | 100 000 Tabletten |
|---|---|---|
| 1. Ethacridinlactat | 200,00 mg | 20,00 kg |
| 2. K 81 1355 A* | 20,00 mg | 2,00 kg |
| 3. Maisstärke | 20,00 mg | 2,00 kg |
| 4. Cellulosepulver | 27,00 mg | 2,70 kg |
| 5. Talkum | 12,00 mg | 1,20 kg |
| 6. Natrium Amylopektin-glykolat | 20,00 mg | 2,00 kg |
| 7. Hochdisperses Silicium-dioxid | 7,00 mg | 0,70 kg |
| 8. Magnesiumstearat | 4,00 mg | 0,40 kg |
| | 310,00 mg | 31,00 kg |

*5-Ethyl-5,8-dihydro-8-oxofuro-[3,2b](1,8)-naphthydrin-7-carbonsäure.

Anstelle von 20 mg der freien Säure der Wirksubstanz K 81 1355 A können alternativ 21,7 mg des Na-Salzes eingesetzt werden. Die Gewichtsdifferenz von 1,7 mg wird an dem Anteil des Cellulosepulvers abgezogen.

Herstellungsverfahren

Ethacridinlactat, K 81 1355 A, Maisstärke, ein Teil des Cellulosepulvers, des Natrium Amylopektinglykolats und des hochdispersen Siliciumdioxids werden gemischt.

Die Mischung wird mit einer geeigneten Tablettenpresse oder einem Walzenstuhl kompaktiert. Die erhaltenen Komprimate werden durch ein Siebe passiert und das entstandene Granulat mit dem Rest des hochdispersen Siliciumdioxids, dem Rest des Cellulosepulvers, dem Rest des Na-Amylopektinglykolats und dem Gesamtanteil an Talkum und Magnesiumstearat gemischt.

Das auf diese Weise entstandene Granulat wird zu Tabletten oder Drageekernen mit einem Endgewicht von 310 mg verpreßt.

Die Drageekerne können filmlackiert oder dragiert werden. Zur Herstellung von Filmtabletten werden dem Fachmann bekannte Filmbildner wie niedrigviskose Hydroxypropylmethylcellulose (z.B. Methocel E 5 cps oder Pharmacoat 606) eingesetzt.

Die Filme können als Lösung in organischen Lösungsmitteln (z.B. Gemisch aus Methylenchlorid und Ethanol) oder Wasser versprüht werden.

Den Abschlußlacken werden bei Bedarf noch Weichmacher und Farbstoffe zugesetzt.

Als geeigneter Abschlußlack empfiehlt sich auch eine wäßrige Dispersion von Eudragit® E der Röhm Pharma GmbH. Rezepturbeispiele können den Firmenprospekten entnommen werden.

Die Gesamtmenge an Lacktrockensubstanz beträgt 10 mg pro Filmtablette.

Bei Bedarf werden die Drageekerne mit einer dem Fachmann bekannten Drageedecke überzogen. Wesentliche Bestandteile einer zum Dragieren verwendeten Suspension sind z.B. Zucker, Gelatine, Arabisches Gummi, Talkum, Calciumcarbonat und Farbpigmente. Als Lösungsmittel bzw. Suspendiermedium für die Hilfsstoffe wird gereinigtes Wasser eingesetzt.

<u>Beispiel 2</u>

| Kapseln | 1 Kapsel | 100 000 Kapseln |
|---|---|---|
| 1. Ethacridinlactat | 200,00 mg | 20,00 kg |
| 2. K 81 1355 A* | 20,00 mg | 2,00 kg |
| 3. Lactose | 86,00 mg | 8,60 kg |

| | | |
|---|---|---|
| 4. Hochdisperses Silicium-dioxid | 2,00 mg | 0,20 kg |
| 5. Talkum | 10,00 mg | 1,00 kg |
| 6. Magnesiumstearat | 2,00 mg | 0,20 kg |
| | 320,00 mg | 32,00 kg |

* Anstelle von 20 mg der freien Säure der Wirksubstanz K 81 1355 A können alternativ 21,7 mg des Na-Salzes eingesetzt werden. Die Gewichtsdifferenz von 1,7 mg wird an dem Anteil der Lactose abgezogen.

Herstellungsverfahren

Die Bestandteile (1) - (6) werden homogen gemischt und in Hartgelatinesteckkapseln Größe 1 angefüllt.

Beispiel 3

| Kapseln | 1 Kapsel | 100 000 Kapseln |
|---|---|---|
| 1. Ethacridinlactat | 200,00 mg | 20,00 kg |
| 2. K 81 1355 A* | 20,00 mg | 2,00 kg |
| 3. Maisstärke modifiziert | 96,00 mg | 9,60 kg |
| 4. Hochdispers4s Silicium-dioxid | 2,00 mg | 0,20 kg |
| 5. Magnesiumstearat | 2,00 mg | 0,20 kg |
| | 320,00 mg | 32,00 kg |

* Einwaage des Na-Salzes entspr. Beispiel 2.

Herstellungsverfahren

Die Bestandteile (1) - (5) werden homogen gemischt und in Hartgelatinesteckkapseln Größe 1 abgefüllt.

PATENTANSPRÜCHE:

1. Arzneimittel zur Behandlung von Diarrhöen, gekennzeichnet durch einen Gehalt an a) einer im Darmlumen antiseptisch wirkenden Substanz und b) einer systemisch antibakteriell wirkenden Substanz.

2. Arzneimittel zur Behandlung von Diarrhöen, gekennzeichnet durch einen Gehalt an

a) Ethacridinlactat 3,9-Diamino-7-äthoxyacridin-lactat, Trypaflavin 3,6-Diamino-N-methyl-acridiniumchlorid, Clioquinol 5-Chlor-8-hydroxy-7-jodchinolin oder Halquinol 5,7-Dichlor-8-hydroxychinolin,

in Mischung mit

b) einer Verbindung der allgemeinen Formel I

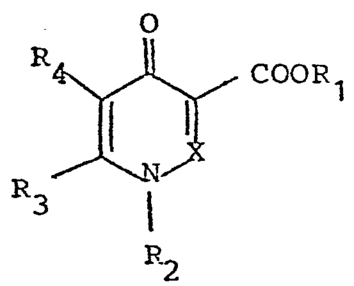

(I)

in der X - CH= oder -N= bedeutet,

$R_1$ Wasserstoff oder einen Aminoalkylrest mit 3 bis 8 Kohlenstoffatomen, und

$R_2$ einen Alkyl- oder Alkoxyrest mit jeweils 1 - 4 C-Atomen. bedeuten,

$R_3$ und $R_4$ zusammen einen an den Pyridonring anellierten aromatischen sechsgliedrigen Ring bilden, der ein oder zwei Stickstoffatome enthalten und durch Fluor, Methyl, den Pyridinrest, Pyrrolidinrest, Piperazinrest, den N-Methylpiperazinrest oder durch weitere Reste substituiert sein kann, die ihrerseits miteinander oder gemeinsam mit $R_2$ einen gegebenenfalls durch Sauerstoff oder Methyl substituierten fünf- oder sechsgliedrigen Ring bilden können, der ein oder zwei Sauerstoffatome oder Schwefel und Stickstoff enthalten kann,

sowie den physiologisch verträglichen Salzen dieser Verbindungen.

3. Arzneimittel nach Anspruch 1 oder 2, dadurch gekennzeichnet, daß als im Darvolumen antiseptisch wirkende Verbindung a) Ethacridinlactat verwendet wird.

4. Arzneimittel nach Anspruch 1, 2 oder 3, dadurch gekennzeichnet, daß die Verbindung der allgemeinen Formel I ein 4-Oxo-1,8-naphthyridin-3-carbonsäure-Derivat der allgemeinen Formel II ist,

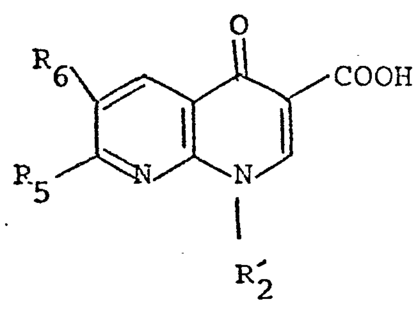

(II)

worin $R_2'$ Methyl oder Äthyl bedeutet, $R_5$ und $R_6$ wechselweise für Wasserstoff oder Alkyl mit 1 oder 2 C-Atomen stehen, oder $R_5$ und $R_6$ gemeinsam einen Furanring bilden.

5. Arzneimittel nach Anspruch 1, 2, 3 oder 4, dadurch gekennzeichnet, daß die Verbindung der allgemeinen Formel I Nalidixinsäure 1-Äthyl-7-methyl-4-oxo-1,4-dihydro-1,8-naphtyridin-3-carbonsäure der Formel III

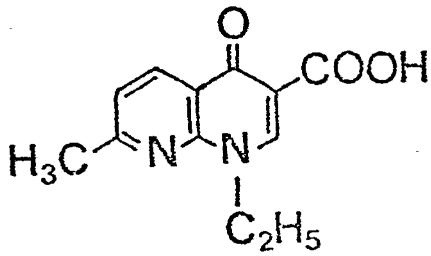

(III)

ist.

6. Arzneimittel nach Anspruch 1, 2, 3 oder 4, dadurch gekennzeichnet, daß die Verbindung der allgemeinen Formel I 5-Äthyl-5,8-dihydro-8-oxofuro(3,2-b)(1,8)-naphthyridin-7-carbonsäure der Formel IV

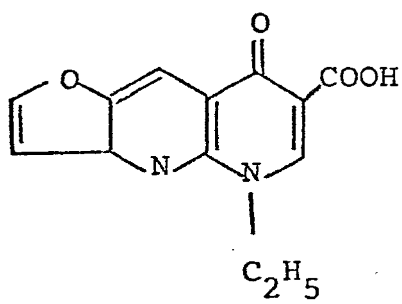

(IV)

ist.

7. Arzneimittel nach Anspruch 1, 2 oder 3, dadurch gekennzeichnet, daß die Verbindung der allgemeinen Formel I ein 4-Chinolon-3-carbonsäure-Derivat der allgemeinen Formel V ist,

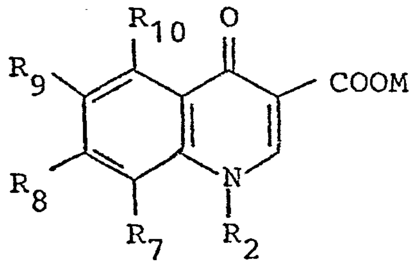

(V)

worin M Wasserstoff, das Kation einer physiologisch verträglichen Base, insbesondere ein Alkali-, Erdalkali- oder Ammonium-Kation, oder eine gegebenenfalls substituierte Alkylgruppe mit 1-8 C-Atomen,

$R_2$ einen Alkyl- oder Alkoxyrest mit jeweils 1-4 C-Atomen,

$R_7$ Wasserstoff, oder einen Alkyl- oder Alkoxyrest, der gemeinsam mit $R_2$ einen sechsgliedrigen, gegebenenfalls ein Sauerstoffatom enthaltenden oder durch Methyl substituierten Ring bildet,

$R_8$ Wasserstoff oder einen Pyridinring, Pyrrolidinring, Piperazinring oder N-Methyl-piperazinring,

$R_9$ Wasserstoff oder Fluor oder $R_8$ und $R_9$ gemeinsam einen fünfgliedrigen Ring mit einem oder zwei Sauerstoffatomen, und

$R_{10}$ Wasserstoff oder gemeinsam mit $R_9$ einen am Stickstoff methylierten Thiazolonring bedeuten.

8. Arzneimittel nach Anspruch 1, 2, 3 oder 7, dadurch gekennkennzeichnet, daß die Verbindung der allgemeinen Formel I bzw. IV Metioxate der Formel VI ist.

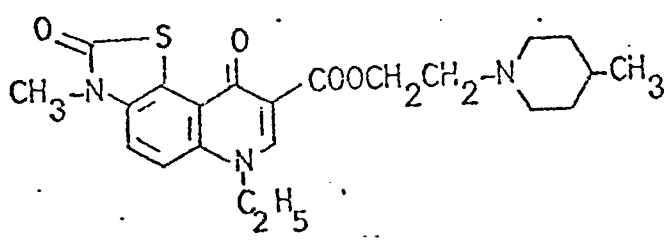

(VI)

9. Arzneimittel nach Anspruch 1, 2 oder 7, dadurch gekennzeichnet, daß die Verbindung der allgemeinen Formel I bzw. IV Norfloxacin der Formel VII ist.

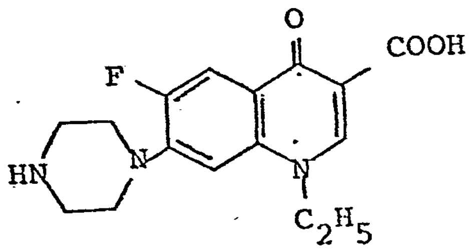

(VII)

10. Arzneimittel nach Anspruch 1, 2, 3 oder 7, dadurch gekennzeichnet, daß die Verbindung der allgemeinen Formel I bzw. IV 9-Fluor-3-methyl-10(4-methyl-1-piperazinyl -7-oxo-2,3-dihydro-7H-pyrido/1,2,3-de/1,4-benzoxazin-6-carbonsäure der Formel VIII ist.

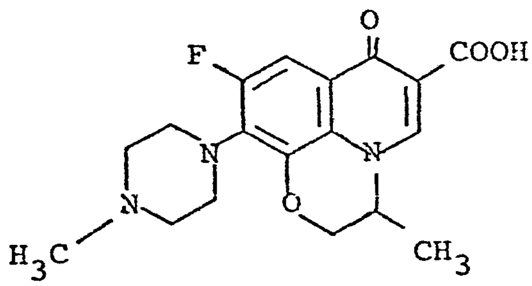

(VIII)

11. Arzneimittel nach Anspruch 1, 2, 3 oder 7, dadurch gekennzeichnet, daß die Verbindung der allgemeinen Formel I bzw. IV Miloxacin 5,8-Dihydro-5-methoxy-8-oxo-2H-1,3-dioxolo(4,5-g)-chinolin-7-carbonsäure der Formel IX.

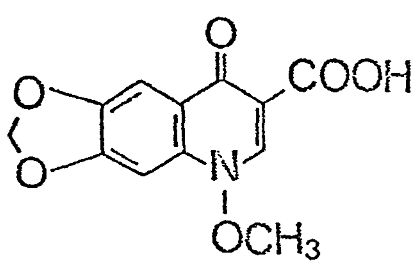

(IX)

ist.

12. Arzneimittel nach Anspruch 1, 2, 3 oder 7, dadurch gekennzeichnet, daß die Verbindung der allgemeinen Formel I bzw. IV, Rosoxacin der Formel X ist.

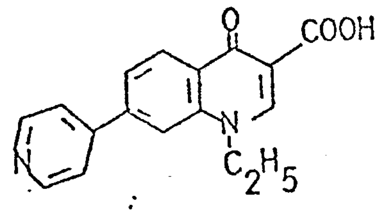

(X)

13. Arzneimittel nach Anspruch 1, 2, 3 oder 7, dadurch gekennzeichnet, daß die Verbindung der allgemeinen Formel I bzw. IV, Oxolinsäure der Formel XI ist.

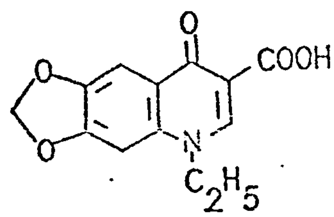

(XI)

14. Arzneimittel nach Anspruch 1, 2, 3 oder 7, dadurch gekennzeichnet, daß die Verbindung der allgemeinen Formel I bzw. IV, Tioxan der Formel XII ist.

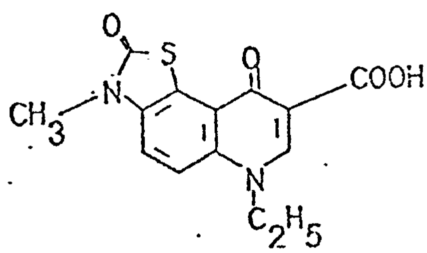

(XII)

15. Arzneimittel nach Anspruch 1, 2, 3 oder 7, dadurch gekennzeichnet, daß die Verbindung der allgemeinen Formel I Flumequin der Formel XIII ist.

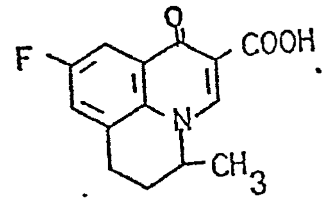

(XIII)

16. Arzneimittel nach Anspruch 1, 2, 3 oder 7, dadurch gekennzeichnet, daß die Verbindung der allgemeinen Formel I Droxacin der Formel XIV ist.

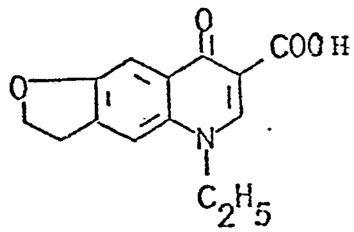

(XIV)

17. Arzneimittel nach Anspruch 1, 2 oder 3, dadurch gekennzeichnet, daß die Verbindung der allgemeinen Formel I Piromidsäure der Formel XV ist.

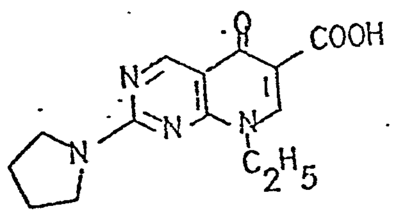

(XV)

18. Arzneimittel nach Anspruch 1, 2 oder 3, dadurch gekennzeichnet, daß die Verbindung der allgemeinen Formel I Piperamsäure der Formel XVI ist.

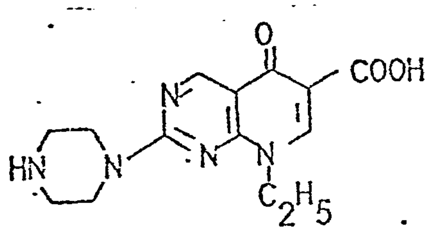

(XVI)

19. Arzneimittel nach Anspruch 1, 2, 3 oder 7, dadurch gekennzeichnet, daß die Verbindung der allgemeinen Formel I Cinoxacin der Formel XVII ist.

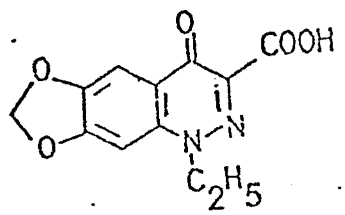

(XVII)

20. Arzneimittel nach Ansprüchen 1-19 , dadurch gekennzeichnet, daß der Gehalt an Ethacridinlactat, Trypaflavin, Clioquinol oder Halquinol 100 - 300 mg beträgt.

21. Arzneimittel nach Ansprüchen 1-19, dadurch gekennzeichnet, daß der Gehalt an Ethacridinlactat oder Trypaflavin 150 - 250 mg beträgt.

22. Arzneimittel nach Ansprüchen 1-21, dadurch gekennzeichnet, daß das Gewichtsverhältnis von Ethacridinlactat, Trypaflavin, Clioquinol oder Halquinol zu einer Verbindung der allgemeinen Formeln I, II, oder V bzw. zu einer Verbindung der Formeln III, IV oder VI bis XVII 1 : 0,4 bis 1 : 5 beträgt.